(19) Europäisches Patentamt
European Patent Office
Office européen des brevets

(11) Veröffentlichungsnummer: **0 264 928**
**A2**

(12) # EUROPÄISCHE PATENTANMELDUNG

(21) Anmeldenummer: **87115416.7**

(22) Anmeldetag: **21.10.87**

(51) Int. Cl.4: **C07D 487/22** , //(C07D487/22,257:00,209:00,209:00,209:00,209:00)

(30) Priorität: **22.10.86 DE 3635820**

(43) Veröffentlichungstag der Anmeldung:
**27.04.88 Patentblatt 88/17**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Franck, Burchard, Prof. Dr.**
**Orléansring 23**
**D-4400 Muenster(DE)**
Erfinder: **Gosmann, Martin**
**Schulte-Bernd-Strasse 18**
**D-4400 Muenster(DE)**

(54) **Vinyloge Porphyrine.**

(57) Die Erfindung betrifft Verbindungen der allgemeinen Formel I

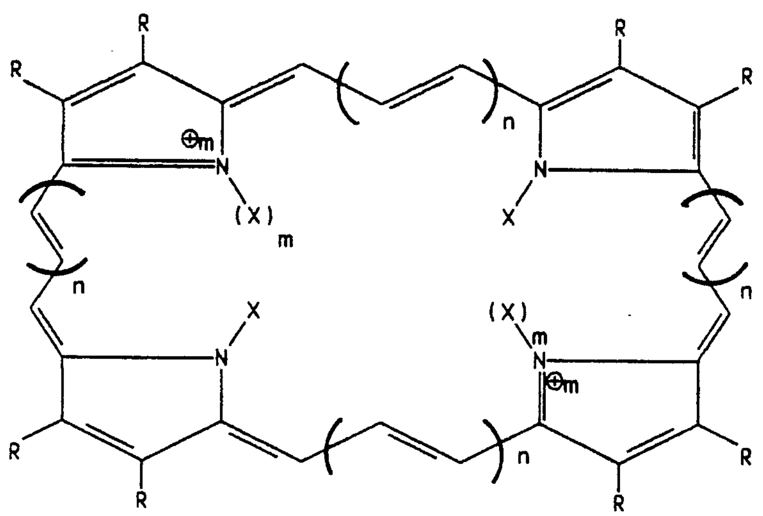

$(2\ A^{\ominus})_m$

(I),

in der die Symbole
n unabhängig voneinander die Zahlen 0, 1 oder 2,
m die Zahlen 0 oder 1,
$A^{\ominus}$ ein Anion, die Reste
X unabhängig voneinander Wasserstoff, Methyl oder Ethyl und die Reste
R unabhängig voneinander Wasserstoff, gegebenenfalls substituiertes Alkyl, Cycloalkyl oder Aryl oder zwei benachbarte Reste R auch eine Alkylengruppe oder zusammen mit den C-Atomen, an die sie gebunden sind, einen ungesättigten oder aromatischen Ring bilden, mit der Maßgabe, daß mindestens ein n von 0 verschieden ist.

EP 0 264 928 A2

Xerox Copy Centre

## Vinyloge Porphyrine

Die Erfindung betrifft Verbindungen der allgemeinen Formel I

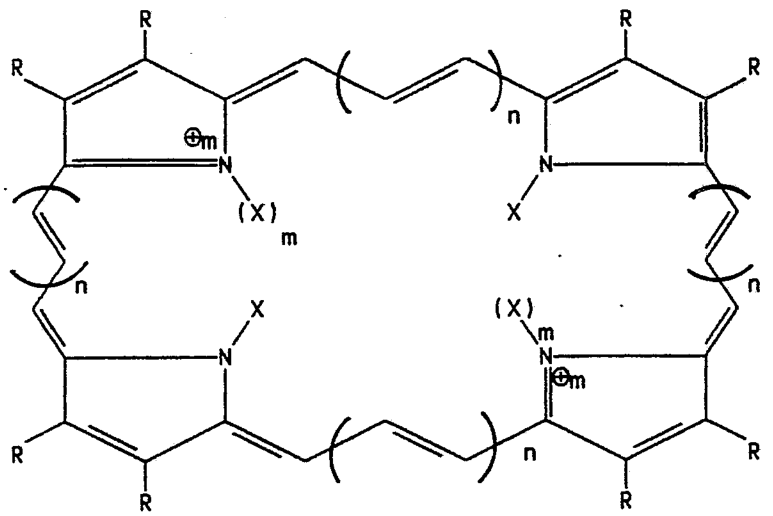

$(2\ A^{\ominus})_m$

(I),

in der die Symbole

n unabhängig voneinander die Zahlen 0, 1 oder 2,

m die Zahlen 0 oder 1,

$A^{\ominus}$ ein Anion, die Reste

X unabhängig voneinander Wasserstoff, Methyl oder Ethyl und die Reste

R unabhängig voneinander Wasserstoff, gegebenenfalls substituiertes Alkyl, Cycloalkyl oder Aryl oder zwei benachbarte Reste R auch eine Alkylengruppe oder zusammen mit den C-Atomen, an die sie gebunden sind, einen ungesättigten oder aromatischen Ring bilden, mit der Maßgabe, daß mindestens ein n von 0 verschieden ist.

Reste R sind z.B.:

$C_1$-bis $C_{20}$-Alkyl, wobei der Alkylrest linear oder verzweigt sein kann, $C_5$-bis $C_7$-Cycloalkyl, $C_7$-bis $C_{10}$-Phenylalkyl, durch $C_1$-bis $C_{12}$-Alkoxycarbonyl substituiertes $C_1$-bis $C_6$-Alkyl oder gegebenenfalls durch Chlor, Brom, Cyan, $C_1$-bis $C_4$-Alkyl oder -Alkoxy substituiertes Phenyl.

Einzelne Reste R sind z.B. Methyl, Ethyl, Propyl, Butyl, Hexyl, Octyl, 2-Ethylhexyl, Decyl, Dodecyl, Octadecyl, Hexadecyl, Cyclohexyl, Benzyl, 2-Phenylethyl, Phenyl, 3-und 4-Chlorphenyl, 3-und 4-Methylphenyl, 3-und 4-Methoxyphenyl, 4-Cyanphenyl, 4-Bromphenyl, $C_1/C_{12}$-Alkoxycarbonylmethyl, $C_1/C_{12}$-Alkoxycarbonylethyl worin $C_1/C_{12}$-Alkoxy z. B. Methoxy, Ethoxy, n-und i-Propoxy, n-und i-Butoxy, Pentoxy, Hexoxy, Octoxy, 2-Ethylhexoxy, Decoxy oder Dodecoxy sein können.

Zur Herstellung der Verbindungen der Formel I kann man Verbindungen der Formel

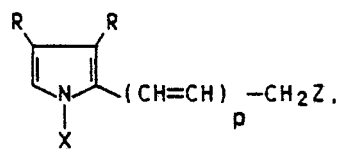

in der

Z Hydroxy, Alkoxy, Acyloxy, Chlor, Brom oder Amino und

p 0, 1 oder 2 sind und X und R die angegebene Bedeutung haben in Gegenwart eines sauren Kondensationsmittels umsetzen und anschließend dehydrieren. Wenn X ≠ H Wasserstoff ist, erhält man dabei die doppelt kationischen Verbindungen.

Als Anionen $A^{\ominus}$ dafür sind beispielsweise Chlorid, Bromid, Jodid, Perchlorat, Tetrachlorozinkat, Hexafluoroantimonat, Hexachloroantimonat, Hexafluorophosphat, Sulfat, Phosphat, Acetat, Trifluoracetat, Benzolsulfonat oder Tosylat zu nennen.

Reste Z sind im einzelnen neben den bereits genannten z.B.: $OCH_3$, $OC_2H_5$, $OC_3H_7$, $OC_4H_9$, $OCOCH_3$, $OCOC_2H_5$ oder $OCOCH_2Cl$.

Einzelheiten der Herstellung können den Beispielen entnommen werden, in denen sich Angaben über Teile und Prozente, sofern nicht anders vermerkt, auf das Gewicht beziehen.

Die Verbindungen der Formel I zeichnen sich durch sehr hohe Extinktionen aus und eignen sich z.B. als Sensibilisatoren für photochemische Reaktionen oder bei der optischen Informationsaufzeichnung.

Von besonderer Bedeutung sind Verbindungen der Formel I, bei denen die Reste R $C_1$-bis $C_4$-Alkyl oder $C_1$-bis $C_8$-Alkoxycarbonylmethyl und n 0 oder 1 sind. Von diesen sind Verbindungen (I) besonders bevorzugt, bei denen n = 1 ist oder zwei der n = 1 und die anderen zwei n = 0 sind.

Beispiel 1

Darstellung des N,N',N'',N''' -Tetramethyl-(26)Porphyrinogens

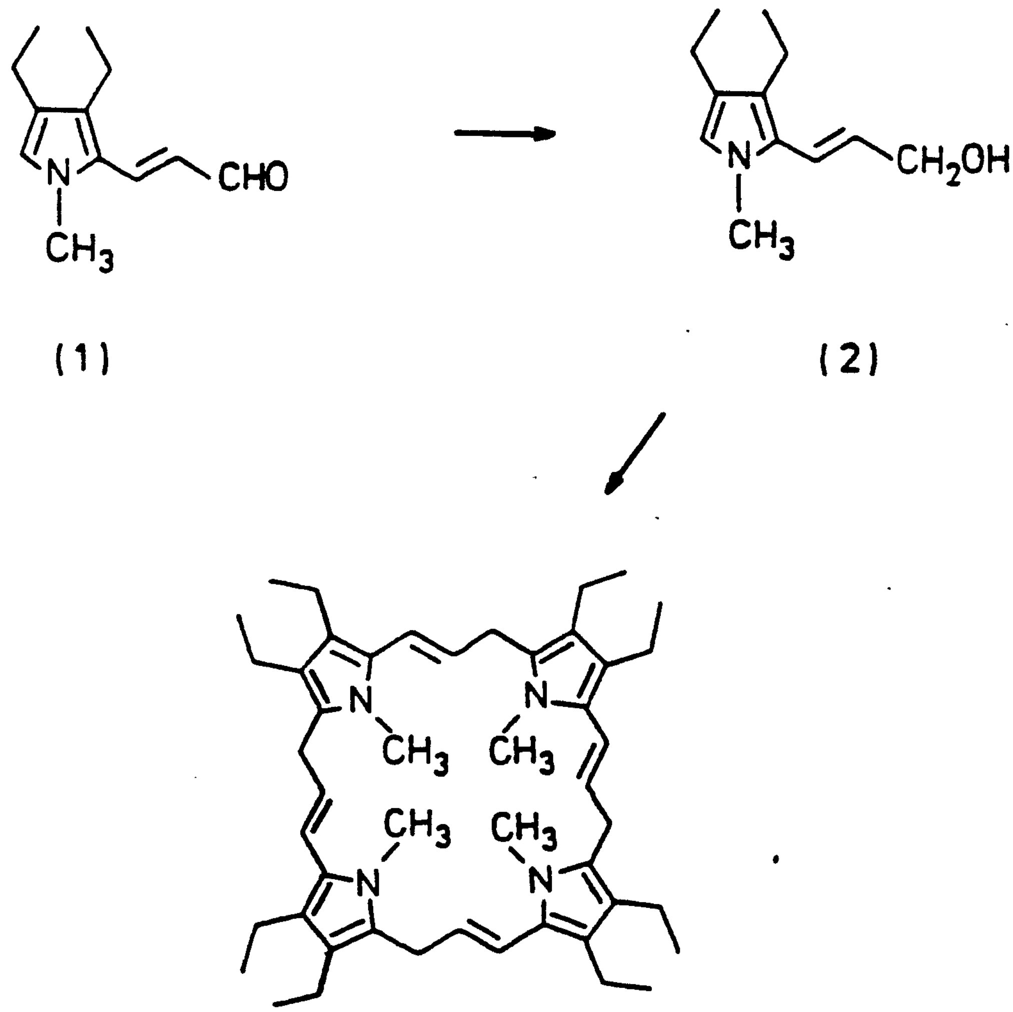

(1)

(2)

(3)

Man löste unter Schutzgas 300 mg (1,47 mmol) des N-Methylpyrrylacroleins (1) in 75 ml absol. Methanol und reduzierte 30 min. mit 300 mg (8 mmol) Natriumborhydrid zu (2). Dann wurde langsam (1,5 h) eine Lösung von 40 mg p-Toluolsulfonsäure in 50 ml Eisessig p.a. zugetropft. Man rührte noch 30 min. und verteilte danach zwischen 100 ml Chloroform und 500 ml Wasser. Die organische Phase wurde nochmals mit Wasser versetzt und der pH-Wert auf pH = 9 eingestellt. Nach dem Abdestillieren des Lösungsmittels wurde das Produkt säulenchromatographisch (60 g Kieselgel, Chloroform) vorgereinigt. Das

so gewonnene Rohprodukt fällte man zweimal aus wenig Methylenchlorid/Methanol um. Es wurde durch eine Mikrofritte abgesaugt, zweimal mit wenig kaltem $CH_3OH/CH_2Cl_2$ = 10:1 und einmal mit Methanol nachgewaschen. Trocknen im Ölpumpenvakuum ergab (3) als farbloses Pulver.

Aubeute: 69-83 mg (25-30 %)

Schmelzpunkt 149-250°C (unter Zers.)

$C_{48}H_{68}N$ (701,1)

Ber. C 82,23 H 9,78 N 7,99

Gef. C 82,24 H 9,77 N 7,93

$C_{48}H_{68}N$ Ber. 700,5444 Gef. 700,5434 (massenspektrometr.)

$^1$H-NMR (300 MHz, $CDCl_3$, ppm bezogen auf $CHCl_3$ mit $\delta$ = 7,27): $\delta$ = 1,08 und 1,11 (2 t, J = 7,5 Hz, 24 H, $CH_2C\underline{H}_3$); 2,41 und 2,50 (2 q, J = 7,5 Hz, je 8 H, $C\underline{H}_2CH_3$); 3,28 (s, 12 H, N-$CH_3$); 3,44 (d, J = 5,5 Hz, 8 H, CH = $CHC\underline{H}_2$); 5,68 (dt, J = 16 und 5,5 Hz, 4H, CH = $C\underline{H}$ $CH_2$); 5,97 (d, J = 16 Hz, 4 H, $C\underline{H}$ = $CHCH_2$);

MS (EI, 70 eV): m/z = 700 (100 %, M+); 685 (4 %, M - $CH_3$); 671 (100 %, M - $C_2H_5$); 525 (8 %, 3/4 M); 350 (56 %, M/2); 335 (17 %, 350 - $CH_3$); 321 (22 %, 350 - $C_2H_5$); 176 und 174 (67 %, 66 %, Monopyrroleinheiten).

IR (KBr, $cm^{-1}$): 2940, 29101 und 2855 (s, aliphat. CH); 1630 (w, CH = CH); 1495 (m, aromat. C = C); 1445 (s, $\delta$-CH); 1372 (s, $\delta$-$CH_3$); 960 (s, trans R-CH = CH-R).

Beispiel 2

N,N',N'',N'''-Tetramethyl-[26]Porphyrin-(3.3.3.3)-bis-trifluoracetat

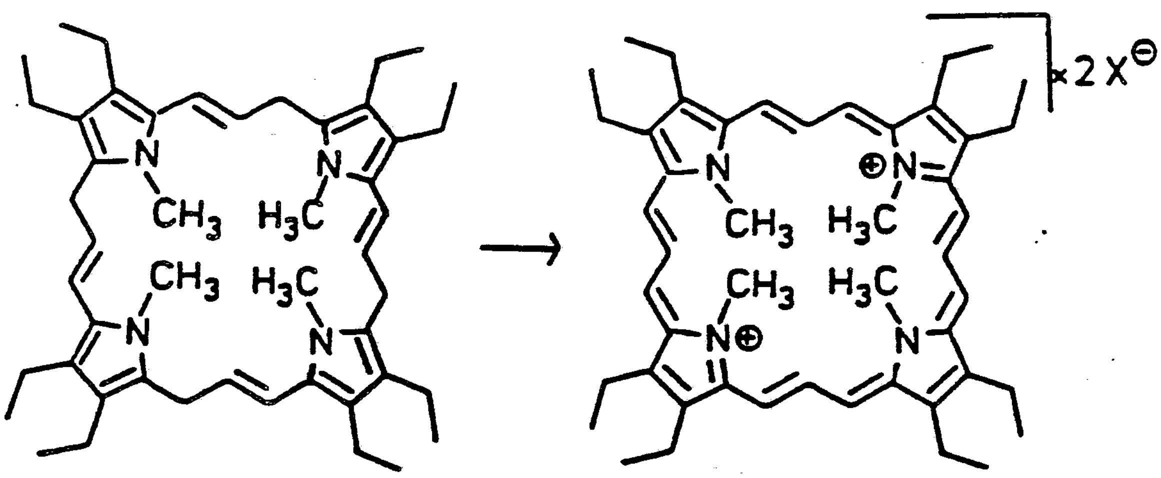

(3) (4)

$X^{\ominus}$ = $CF_3COO^{\ominus}$

105,2 mg (0,15 mmol) des N,N',N'',N'''-Tetramethyl-[26]Porphyrinogens (3) (aus Beispiel 1) wurden mit 200 mg (0,60 mmol) Polymerbase (Diethylaminomethylpolystyrol, 3,0-3,2 mAequivalente Base/g Harz) in 150 ml dest. Chloroform unter Schutzgas gerührt und dann langsam 12,85 ml (0,45 mmol) einer Lösung von Brom in Tetrachlorkohlenstoff (560 mg Brom/100 ml) zugetropft. Man ließ die Mischung über Nacht weiterrühren, filtrierte die Polymerbase ab und engte die Lösung am Rotationsverdampfer ein. Der metallisch grün glänzende Rückstand wurde einer zweimaligen chromatographischen Reinigung an Kieselgel (15 g) unterworfen (Laufmittelsystem: Acetonitril/Trifluoressigsäure = 250:1). Die vereinigten Produktfraktionen der zweiten Säulentrennung wurden nach Zugabe von 50 ml Chloroform mit dest. Wasser neutral gewaschen und noch 10 Min. mit 100 mg Polymerbase gerührt. Der nach dem Abfiltrieren und Abdampfen des Lösungsmittels anfallende Rückstand lieferte aus Methylenchlorid/Ether das Bistrifluoracetat des [26]-Porphyrins (4) als analysenreines, grünes Pulver, welches noch 3 h im Ölpumpenvakuum getrocknet wurde. Zersetzung ohne Schmelzen. Ausbeute: 48,5 mg (35 %)

DC: (Kieselgel; $CH_3CN/CF_3COOH$ = 250:1) $R_f$ = 0,20

$(C_{48}H_{64}N_4^{2+})(CF_3COO^-)_2$ (923,1)
Ber.: C 67,66 H 6,99 N 6,07 F 12,35
Gef.: C 67,56 H 7,06 N 6,15 F 12,2 (Br 0,27)
UV/VIS (Chloroform): $\lambda_{max}$ ($\epsilon$) = 547 (909600) nm
IR (KBr, $cm^{-1}$): 2955, 2920 und 2860 (m, aliphat. CH);
1675 (s, CO/Trifluoracetat).
$^1H$-NMR (300 MHz, $CDCl_3$): $\delta$ = -11,64 (t, J = 13,7 Hz, 4 H, innere Brücken-H); -9,09 (s, 12 H, N-$CH_3$); 2,43 (t, J = 7,7 Hz, 24 H, Ethyl-$CH_3$); 4,99 (q, J = 7,7 Hz, 16 H, Ethyl-$CH_2$); 13,67 (d, J = 13,7 Hz, 8 H, äußere Brücken-H).
FD-MS ($CH_3CN$; 8 kV, 8-15 mA): m/z = 697 (100 %, $C_{48}H_{64}N_4$ = M - 2 $CF_3COO^-$); 695 (25 %, M - 2 $CF_3COOH$).

Löslichkeitseigenschaften:

Die Verbindung löst sich in organischen Lösungsmitteln wie Chloroform, Methylenchlorid, Aceton, Acetonitril, Methanol, nicht aber in Ether und unpolareren Lösungsmitteln.

Beispiel 3

[22]Porphyrin-(1.3.1.3)-octaessigsäureethylester (7)

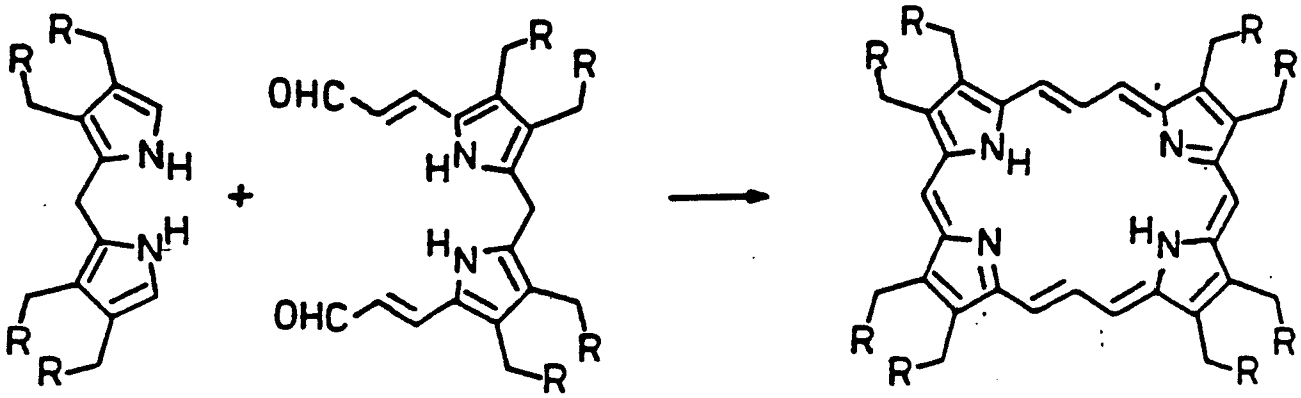

(5) (6) (7)

R = $COOCH_2CH_3$

24,5 mg (0,05 mmol) des Dipyrrylmethans (5) und 29,9 mg (0,05 mmol) des Dipyrryldiacroleins (6) wurden in 150 ml absol. Methylenchlorid 20 Min. unter Argon gerührt, bevor man langsam 1 ml 33 %igen Bromwasserstoff in Eisessig zusetzte. Nach einstündigem Rühren bei Raumtemperatur tropfte man 1,43 ml (0,05 mmol) einer Lösung von Brom in Tetrachlorkohlenstoff (560 mg Brom/100 ml) zu und ließ 2,5 h weiterrühren. Die grüne Reaktionsmischung wurde zunächst mit 2 N Natriumhydrogencarbonatlösung, dann mit Wasser neutral gewaschen, über Natriumsulfat getrocknet und das Lösungsmittel abgedampft. Den Rückstand reinigte man durch Kieselgelfiltration (5 g, Methylenchlorid/Ether = 4:1) und Umfällen aus Methylenchlorid/Ether. Dunkler olivgrüner Feststoff, der ohne Schmelzen zersetzt. Ausbeute: 28 mg (53 %) Porphyrin (7)
$(C_{56}H_{66}N_4O_{16}$ (1051,2)
Ber.: C 63,99 H 6,33 N 5,33
Gef.: C 64,15 H 6,36 N 5,26
UV/VIS (Chloroform): $\lambda_{max}$ ($\epsilon$) = 485 (426000) nm, 505 (342500) nm.

(Chloroform/1 % Trifluoressigsäure): $\lambda_{max}$ ($\epsilon$) = 469 (1068000) nm.
IR (KBr, $cm^{-1}$): 2970, 2925 (w, aliphat. CH);
1723 (s; CO-Ester):
$^1$H-NMR (300 MHz, $CDCl_3$, ppm): $\delta$ = -8,19 (t, J = 13,3 Hz, 2 H, innere Trimethin-Brücken-H); 1,27 und 1,31 (2 t, J = 7,1 Hz, je 12 H, Ester-$CH_3$); 4,32 (2 q, J = 7,1 Hz, je 8 H, Ester-$CH_2$); 5,37 und 5,48 (2 s, je 8-H, 22 Porphyrin-$CH_2$); 10,59 (s, 2 H, Monomethin-Brücken-H); 11,91 (d, J = 13,3 Hz, 4 H, äußere Trimethin-Brücken-H).
MS (EI; 70 eV; 3 kV Beschleunigungsspannung): m/z = 1051 ± 1 (16 %), $M^+$).
FD-MS ($CHCl_3$; 2 kV; 8-15 mA): m/z = 1050 ± 1 (100 %, M+).
Die Verbindung löst sich in Chloroform, Methylenchlorid und Methanol.

## Ansprüche

1. Verbindungen der allgemeinen Formel I

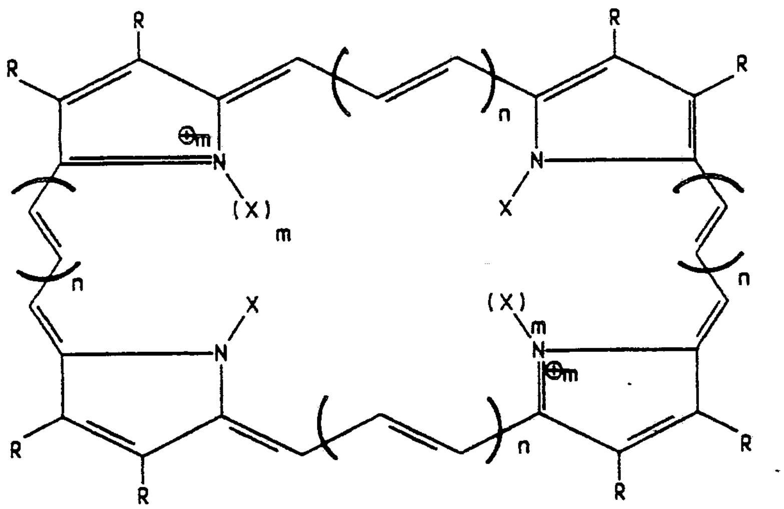

(2 A⊖)$_m$

(I),

in der die Symbole
n unabhängig voneinander die Zahlen 0, 1 oder 2,
m die Zahlen 0 oder 1,
A⊖ ein Anion, die Reste
X unabhängig voneinander Wasserstoff, Methyl oder Ethyl und die Reste
R unabhängig voneinander Wasserstoff, gegebenenfalls substituiertes Alkyl, Cycloalkyl oder Aryl oder zwei benachbarte Reste R auch eine Alkylengruppe oder zusammen mit den C-Atomen, an die sie gebunden sind, einen ungesättigten oder aromatischen Ring bilden, mit der Maßgabe, daß mindestens ein n von 0 verschieden ist.

2. Verbindungen gemäß Anspruch 1, bei denen die Reste R $C_1$-bis $C_4$-Alkyl oder $C_1$-bis $C_8$-Alkoxycarbonylmethyl und n 0 oder 1 sind.